# EUROPEAN PATENT SPECIFICATION

(11) **EP 2 444 091 B1**
(45) Date of publication and mention of the grant of the patent: **21.05.2014**
(21) Application number: 10789761.3
(22) Date of filing: 18.06.2010
(51) Int. Cl.: A61K 31/568, A61K 36/58, A61P 25/28

(54) **USE OF TOOSENDANIN OR MELIA AZEDARACH EXTRACTS FOR PREVENTING OR TREATING DEMENTIA**
VERWENDUNG VON TOOSENDANIN- ODER MELIA AZEDARACH-EXTRAKTEN ZUR PRÄVENTION ODER BEHANDLUNG VON DEMENZ
UTILISATION DE TOOSENDANINE OU D'EXTRAITS DE MELIA AZEDARACH POUR PRÉVENIR OU TRAITER LA DÉMENCE

(30) Priority: 19.06.2009 KR 20090054949
(43) Date of publication of application: 25.04.2012
(73) Proprietor: Ildong Pharm Co., Ltd., Seoul 137-733 (KR)
(72) Inventor: CHOI, Jin Sung, Hwaseong-si Gyeonggi-do 445-788 (KR); YOO, Jong Su, Suwon-si Gyeonggi-do 443-470 (KR); YOU, Young Sang, Suwon-si Gyeonggi-do 441-400 (KR); KIM, Young Jae, Gyeonggi-do 445-160 (KR); PARK, Eun Kyung, Yongin-si Gyeonggi-do 446-525 (KR); RYU, Jung Su, Yongin-si Gyeonggi-do 448-120 (KR); YEON, Seung Woo, Yongin-si Gyeonggi-do 446-745 (KR); KANG, Jae Hoon, Seoul 135-280 (KR)
(74) Representative: Gulde & Partner
(86) International application number: PCT/KR2010/003971
(87) International publication number: WO 2010/147434

(56) References cited:
- KR-A- 20070 012 038
- KR-A- 20100 011 453
- KR-B1- 100 345 226
- KR-B1- 100 750 879
- US-A- 4 536 496

## Description

### Technical Field

This application claims priority from and the benefit of Korean Patent Application No. 10-2009-0054949, filed on June 19, 2009, which is hereby incorporated by reference for all purposes as if fully set forth herein.

The present invention relates to the use of toosendanin or Chinaberry tree (Melia azedarach, Melia toosendan) extracts for preventing or treating dementia. More particularly, the present invention relates to a pharmaceutical composition for use in preventing or treating dementia comprising toosendanin or its pharmaceutically acceptable salt thereof.

### Background Art

Dementia is a syndrome that gradually progresses in the long term, a serious disorder in memory faculty, concentration, language and recognition due to damage or loss of nerve cells, finally resulting in the loss of mental capacity and social activity. A representative disease causing dementia is Alzheimer's disease. Alzheimer's disease is divided into a sporadic type and a familial type according to its cause. The sporadic type of Alzheimer's disease, with unknown causes, occurs in 80~90% or more of Alzheimer's disease patients, and mainly occurs in older people (after age 65). The other type of Alzheimer's disease is mainly caused by a mutation of genes such as an amyloid precursor protein (hereinafter, referred to as an APP), and presenilin (PS)-1 and 2, and always occurs in younger people (before age 65). It is recognized that symptoms of Alzheimer's is mainly caused by a beta amyloid (hereinafter, referred to as Aβ) forming a senile plaque disease outside of nerve cells of brain tissues, and a hyperphosphorylated tau protein forming a paired helical filament within nerve cells. It was reported that Aβ is a protein consisting of 40-42 amino acids, which is formed through APP's amino acid sequence specific metabolism by some proteases, that is, β- and γ-secretases, and causes neurotoxicity, and synapse loss and inflammation by being aggregated itself (Cappari et al, Neurochem Res, 2008, 33:526-532). Also, it was reported that a tau protein performs a role of maintaining the shape and the structure of a cell by being bound to a microtubule, and a hyperphosphorylated tau protein is separated from the microtubule, destroys the microtubule, and forms neurofibrillary tangles, inducing a neuronal cell death (Iqbal et al, Acta Neuropathol, 2009, in print). It is known that the number of patients suffering Alzheimer's disease is about 26 million all over the world (in 2008), and also it is expected that the number will reach about 100 million or more in 2050 according to the increase in an elderly population (Carlsson, J Alz Dis, 2008, 15:327-338). However, the development of a fundamental therapeutic agent has been very slowly conducted. Examples of currently available drugs include acetylcholine esterase activity inhibitors (e.g., Aricept, Exelon, Reminyl) for fixedly maintaining the concentration of acetylcholine (neutrotransmitter related to memory), and an NMDA receptor antagonist (e.g., Memantine) for inhibiting a neuronal cell death caused by Ca²⁺. However, these are used only for lessening the progress of symptoms. Thus, it is urgently required to develop a therapeutic agent showing a fundamental therapeutic effect.

Chinaberry tree (Melia azedarach, Melia toosendan) is classified into Meliaceae family and Melia genus. Its velamen or bark referred to as Meliae cortex and its fruit referred to as Toosendan Fructus. They have been mainly used as anthelmintics since ancient times (in China), and also shows antibacterial, diuresis, and antipyretic effects. Also, according to records in Dongyibogam (Korean Traditional Medical Book), they can be used for treatment of scrotal hernia (swelling or pain of reproductive organ/testicle, and disorder of urine and feces). It was reported that the anthelmintic effect of Meliae cortex or Toosendan Fructus of melia azedarach is obtained by triterpenoid-based toosendanin (or azedarachin, C₃₀H₃₈O₁₁, molecular weight 574, Wang and Yen, J Tradit Chin Med 1959, 262:46-49) because the toosendanin selectively inhibits intake and differentiation of an insect (Zhang et al, Acta Northwe Uni Agricult Sin, 1993, 21:1-5). Besides the Chinaberry tree, Cedrela sinensis is classified into the same Meliaceae family contains the toosendanin. Its root/stem bark, that is, cedrelae cortex, is used as anthelmintic, and antidiarrhotica, and therapeutic agents for shigellosis and leukorrhea.

Besides, toosendanin was reported to induce nerve cell differentiation (Tang et al, Neurosci Res, 2003, 45:225-231) and cancer cell apoptosis (Shi and Tang, Chin J Neurosci, 2004, 20:461-465). Also, there are domestic and overseas patents related to the use of the Chinaberry, including cancel cell cytotoxicity (Korean Registered Patent No. 10-0112032), a whitening and anti-wrinkling effect (Korean Registered Patent No. 10-0345226, and US Registered Patent No. 06,866,856), an insecticidal effect (Korean Registered Patent No. 10-0112657, and US Registered Patent No. 06,372,239), and an allergy prevention effect (Korean Registered Patent No. 10-0750879 B1). Furthermore there is described a composition for treating cancer, containing an extract from the leaves of *Melia azedarach L.* (US patent application No. 4,563,496 A). However, there is no research on toosendanin's effect on dementia at all.

### Disclosure

### Technical Problem

The inventors of the present invention researched a novel material for treating dementia, and found that an extract of chinaberry tree's fruit (that is, Toosendan Fructus) including toosendanin, and toosendanin separated from the extract facilitate the production of APPα showing a protective effect of a nerve cell through metabolism control of APP, and inhibit the production of Aβ, thereby improving a memory in a dementia animal model. Then, they completed this invention based on the finding.

Accordingly, an object of the present invention is to provide the use of toosendanin or its pharmaceutically acceptable salt for preventing or treating dementia.

Another object of the present invention is to provide the use of an extract of chinaberry tree for preventing or treating dementia.

### Technical Solution

To achieve an object of the present invention, the present invention provides a pharmaceutical composition for use in preventing or treating dementia comprising toosendanin or a pharmaceutically acceptable salt thereof as an active ingredient.

To achieve another object of the present invention, the present invention provides a use of toosendanin or a pharmaceutically acceptable salt thereof for preparing an agent for preventing or treating dementia.

To achieve still another object of the present invention, the present invention provides a pharmaceutical composition for use in preventing or treating dementia comprising chinaberry tree (Melia azedarach or Melia toosendan) extract as an active ingredient.

To achieve another object of the present invention, the present invention provides a use of chinaberry tree (Melia azedarach or Melia toosendan) extract for preparing an agent for preventing or treating dementia.

Hereinafter, the present invention will be described in detail.

The used Toosendanin of the present invention represented by Formula 1 is known to show an insecticidal effect, cancer cell cytotoxicity, and whitening and anti-wrinkling effects, and also shows a dementia prevention/treatment effect. The effect of the toosendanin for preventing or treating dementia has been known first by the present invention. 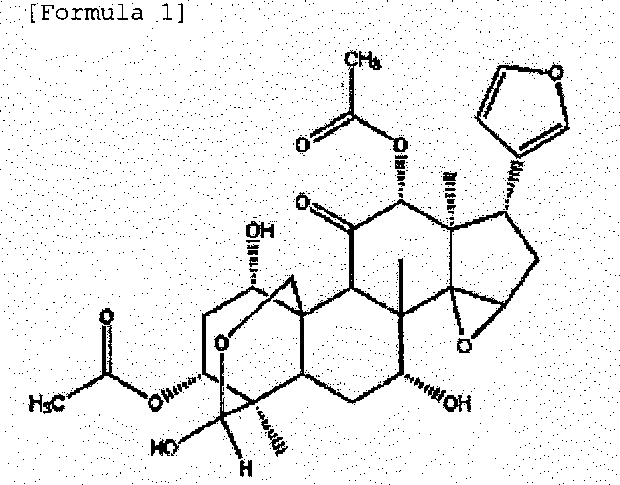 C₃₀H₃₈O₁₁ (molecular weight 574. 62)

Dementia represents a disease causing acquired cognitive dysfunction and personality change, in spite of normal intellectual level in a growth period. In dementia, a cranial nerve is destroyed by various causes, which causes overall disorders in mental functions, such as memory disorder, language disorder, urine/ feces incontinence, paranoia, and aphasis. Furthermore, the progress of dementia may be accompanied by psychological symptoms such as melancholia, personality disorder, and aggression. In the medical field, aging and heredity have been spotlighted as causes of the dementia. However, an exact cause of the disease and a treatment method has not been found out yet. Diseases classified into dementia include, but are not limited to, Alzheimer dementia, cerebrovascular dementia, senile dementia, and dementia by head injuries.

The composition for use according to the present invention comprising toosendanin or its pharmaceutically acceptable salt as an active ingredient may comprise any extract as long as the extract comprises toosendanin as an active ingredient. For example, the extract may be a *Cedrela sinensis* extract or chinaberry tree extract, but the present invention is not limited thereto. Preferably, it may be an extract of chinaberry tree (Melia azedarach or Melia toosendan), and most preferably it may be an extract of toosendan fructus (*Chinaberry's* fruit) or Meliae cortex (velamen or bark of Chinaberry). Two species of chinaberry tree (Melia azedarach and Melia toosendan) are classified into Meliaceae family and in Melia genus. Its velamen or bark (referred to as Meliae cortex) and its fruit (Toosendan Fructus) have been mainly used as anthelmintics since ancient times (in China), and also shows antibacterial, diuresis, and antipyretic effects. Also, according to records in Dongyibogam, they can be used for treatment of scrotal hernia (swelling or pain of reproductive organ/testicle, and disorder of urine and feces). Chinaberry (Melia azedarach or Melia toosendan) extract of the present invention may be extracted and prepared from all parts of Chinaberry, such as fruits, leaves, stems, roots, and preferably may be prepared from fruits, root barks or stem bark.

The used chinaberry tree (Melia azedarach or Melia toosendan) extract of the present invention may be prepared by a solvent extraction method known in the art. As an extraction solvent, for example, any one selected from water, the group including organic solvents such as C1 to C6 alcohol such as ethanol (ethyl alcohol) and methanol, ethyl acetate, dichloromethane, chloroform, n-hexane, diethyl ether, acetone, benzene, or a mixed solvent thereof may be used. Preferably, water or C1 to C6 alcohol may be used for extraction. When the solvent extraction method is used for preparing an extract, hot water extraction, ultrasonic extraction, and reflux extraction may be used. Most preferably, the used chinaberry tree extract of the present invention may be extracted and prepared by using methanol as a solvent. The ratio of chinaberry tree and methanol in the extraction is not particularly limited, but methanol may be added to chinaberry tree in an amount of 3~15 times as much as the weight of chinaberry tree. Preferably, in order to increase the extraction efficiency, methanol may be added to chinaberry in an amount of 5~10 times as much as the weight of chinaberry. The temperature for the extraction may preferably range from 10 to 30°C under atmospheric pressure. Also, the time for the extraction may vary according to the extraction temperature, but may range from 48 to 96 hours, and preferably may be 72 hours. When the extraction time is short, the extraction of a chinaberry component is insufficient. On the other hand, when the extraction time is too long, the extraction yield is lowered by evaporation. Also, in order to increase the extraction efficiency, preferably, chinaberry may be grinded by a grinder, and a methanol extracting step may be repeated twice so as to additionally extract a residual component.

The extract extracted by methanol may be fractionated by a method known in the art in order to remove impurities and increase the concentration of an active ingredient. In order to increase the efficiency of fractionation, the extract may be vacuum-concentrated. As a solvent for the fractionation, any one selected from the group including organic solvents such as C1 to C6 alcohol such as ethanol and methanol, ethyl acetate, dichloromethane, chloroform, n-hexane, diethyl ether, acetone, benzene, or a mixed solvent thereof may be used. Preferably, ethyl acetate may be used for the fractionation. A fraction may be used as it is, or may be re-fractionated. In re-fractionation, preferably, a mixed solvent of water, methanol, and n-hexane may be used.

In an Example of the present invention, toosendan fructus grinded into small pieces was repeatedly extracted twice in methanol in a volume 5 times larger with respect to the weight of toosendan fructus for 3 days, and an extract was filtered, vacuum-concentrated, suspended in water, and treated with ethyl acetate in the same volume. Then, an ethyl acetate fraction was collected and vacuum-concentrated. The concentrated fraction was added with 90~95% methanol and n-hexane in the same volume, followed by stirring. Then, a methanol fraction was collected, and vacuum-concentrated, and a final extract was obtained (See Example 1).

The used toosendanin of the present invention may be separated/purified from a natural material, commercially bought, or prepared by a chemical synthesis method known in the art.

Preferably, the used toosendanin of the present invention may be separated/purified from a natural material. More preferably, it may be separated/purified from *Melia toosendan*, and most preferably, it may be separated/purified from toosendan fructus (chinaberry's fruit) or Meliae cortex (bark of a root or a stem of *Melia toosendan*)*.* A method for obtaining an extract containing toosendanin from *Melia toosendan* is the same as described above. Then, the separation of toosendanin from the extract may be carried out according to a chromatography separation method known in the art. For example, through fractionation using silica column chromatography, and high performance liquid chromatography (HPLC), an active ingredient may be purified.

In an Example of the present invention, chinaberry tree (Melia azedarach or Melia toosendan) extract was fractionated into 8 fractions by 60 mesh silica resin while the mixing ratio of methanol was gradually increased (from 100% dichloromethane: 0% methanol to 0% dichloromethane: 100% methanol). Then, the fractions were obtained. Then, a fraction having the highest activity (dichloromethane: methanol =25:1) was fractionated through octadecyl-silylated silica resin while the concentration of methanol was varied. Then, a fraction having the highest activity was secured, and re-fractionated under the same condition by changing the methanol concentration. Then, a fraction having the highest activity was secured, and through fractionation of HPLC, toosendanin was purified (See Example 2). The final fraction was identified as toosendanin through an NMR spectroscopy analysis and a mass spectroscopy.

Toosendanin as an active ingredient within the composition for use according to the present invention or the chinaberry tree (Melia azedarach or Melia toosendan) extract inhibits the activity of β-secretase, and promotes the activity of α-secretase. Finally, it has an activity for inhibiting the production of a beta amyloid. This was found out first by the inventors of the present invention.

Alzheimer's disease is mainly caused by Aβ and a tau protein, which form a senile plaque and a nerve fiber outside and inside, respectively, of nerve cells. This causes atrophy of a brain tissue, and memory disorder. From among Aβ and tau, Aβ is closely connected to direct death of a nerve cell. In Alzheimer's disease, it is assumed that activation in the production pathway from APP to Aβ induces an initial symptom by a certain reason, and then, a tau protein is related to acceleration of the disease (Small and Duff, Neuron, 2008, 5:591-598). Accordingly, it is thought that for prevention or treatment of Alzheimer's disease, first, the blocking of an Aβ production pathway is important. APP is cleaved into C-terminal fragment β (CTFβ) including an amino acid sequence of APPβ and Aβ by β-secretase, and then through γ-secretase's action on CTFβ, ACID (APP intracellular domain) and Aβ are formed. Thus, the inhibition of activity of β-secretase may be one method for preventing or treating Alzheimer's disease. Also, α-secretase competitively decomposes APP, with respect to β-secretase, thereby inhibiting the production of Aβ. By α-secretase, the production of APPα (one of metabolites of APP) is induced. Accordingly, the improvement of the activity of α-secretase may be another method for preventing or treating Alzheimer's disease.

The composition for use according to the present invention or toosendanin as an active ingredient within the composition for use according to the present invention is an Aβ production inhibitor for effectively blocking the production pathway of Aβ, and promoting the production of APPα showing a protective effect of a nerve cell, which corresponds to the above described object of development of an agent for treating Alzheimer's disease.

In an Example of the present invention, it was found through a cell experiment by western blot that chinaberry tree extract or toosendanin within the composition for use according to the present invention promotes the production of APPα, and inhibits the production of APPβ (see Example 5-1), and also, it was found through a cell experiment by a beta amyloid quantitative analysis kit that they inhibit the production of a beta amyloid (see Example 5-2).

In an Example of the present invention, it was found through an animal experiment by a beta amyloid quantitative analysis kit that chinaberry tree extract or toosendanin within the composition for use according to the present invention reduces the level of a beta amyloid in a dementia animal model (See Example 8).

In an Example of the present invention, it was found that chinaberry tree extract or toosendanin within the composition for use according to the present invention treats memory/cognitive dysfunction (one of main symptoms of dementia) in a dementia animal model, through a Y-maze test, a water maze test, and a passive avoidance test on a dementia-induced rat administered with the composition for use according to the present invention (See Example 7).

Accordingly, it was determined that the composition for use according to the present invention shows a significant effect on preventing or treating dementia while effectively blocking an Aβ production pathway and promoting the production of APPα showing a protective effect of a nerve cell.

In another Example of the present invention, by using ethyl alcohol (ethanol) with various concentrations as an extraction solvent, chinaberry tree extract within the composition for use according to the present invention was obtained, and toxicities and efficacies of respective extracts were compared with each other. As a result, when the used chinaberry tree extract of the present invention was extracted by ethyl alcohol as an extraction solvent, it generally shows a high efficacy irrespective of the concentration of ethyl alcohol. Especially, when the content of ethyl alcohol of an extraction solvent was adjusted to 30% to 50%, it was determined that it is possible to secure the used chinaberry tree extract of the present invention with a high efficacy and a highly lowered toxicity (See Example 9).

Accordingly, the present invention provides a pharmaceutical composition for use in preventing or treating Dementia comprising toosendanin or a pharmaceutically acceptable salt thereof as an active ingredient. Also, the present invention provides a pharmaceutical composition for use in preventing or treating Dementia comprising chinaberry tree (Melia azedarach or Melia toosendan) extract as an active ingredient. A pharmaceutical composition for use according to the present invention may comprise 0.001 to 99.999 weight% of the chinaberry tree (Melia azedarach or Melia toosendan) extract or toosendanin or a pharmaceutically acceptable salt thereof and the rest may be a pharmaceutically acceptable carrier.

Toosendanin used in the present invention may be used itself or in the form of a pharmaceutically acceptable salt. As used herein, the phrase "pharmaceutically acceptable" means that the components present in the composition are physiologically acceptable and usually do not invoke allergic or similar reactions when administered to humans. Preferably, the salt may be an acid addition salt formed from a pharmaceutically acceptable free acid. The free acid may be an organic or inorganic acid. The organic acid includes but is not limited to citric acid, acetic acid, lactic acid, tartaric acid, maleic acid, fumaric acid, formic acid, propionic acid, oxalic acid, trifluoroacetic acid, benzoic acid, gluconic acid, methanesulfonic acid, glycolic acid, succinic acid, 4-toluenesulfonic acid, glutamic acid and aspartic acid. And, the inorganic acid includes but is not limited to hydrochloric acid, bromic acid, sulfuric acid and phosphoric acid.

A pharmaceutical composition for use according to the present invention may comprise toosendanin or a pharmaceutically acceptable salt thereof or chinaberry tree (Melia azedarach or Melia toosendan) extract alone or further comprise pharmaceutically acceptable carriers, exipients or diluents.

. As used herein, the phrase "pharmaceutically acceptable" means that the components present in the composition are non-toxic composition which is physiologically acceptable and usually do not invoke allergic reactions such as gastroenteric disorders, dizziness or similar reactions when administered to humans.

A pharmaceutically acceptable carrier, for example, carriers for the parenteral or oral preparations may be comprised. The carriers for the oral preparations may comprise lactose, starch, cellulose derivatives, magnsium stearate, stearic acid. In addition, they may comprise various drug delivery materials for administration of peptide agents. The carriers for the parenteral preparations may comprise water, suitable oil, saline, aqueous glucose and glycol. They may further comprise stabilizers and preservatives. The examples of the stabilizers may be antioxidants such as sodium hydrogen sulfite, sodium sulfite, and ascorbic acid. The examples of the preservatives may be benzalkonium chloride, methyl- or prophyl-paraben, and chlorobutanol. A pharmaceutical composition for use according to the present invention may further comprise lubricants, wetting agents, sweetening agents, flavors, emulsifying agents and suspension agents. The list of pharmaceutically acceptable carriers are disclosed in Remington' s Pharmaceutical Sciences, 19th ed., Mack Publishing Company, Easton, PA, 1995.

A pharmaceutical composition for use according to the present invention comprising chinaberry tree (Melia azedarach or Melia toosendan) extract, toosendanin or a pharmaceutically acceptable salt thereof as an active ingredient may comprise effective amount of chinaberry tree (Melia azedarach or Melia toosendan) extract, toosendanin or a pharmaceutically acceptable salt thereof alone or further comprise pharmaceutically acceptable carriers. As used herein, "pharmaceutically acceptable amount" refers the mount showing more reaction than negative control and preferably, it refers the sufficient amount to treat or prevent Dementia. As used herein, Dementia may be Alzheimer's disease, cerebrovascular dementia, senile dementia and dementia caused by head injury.

A composition for use according to the present invention may be administered to mammals as well as human by various routes. For example, it may be administered by orally or parenterally. For parenteral administration, it may be administered by, but not limited thereto, intravenous, intramuscular, intraarterial, intramarrow, subdural, intracardiac, intracutaneous, subcutaneous, intraperitoneal, intranasal, gastrointestinal tracts, parenteral, sublingual or rectum.

A pharmaceutical composition for use according to the present invention may be prepared into a formulation for oral administration or parenteral administration, as described above.

In case of the formulation for oral administration, the composition for use according to the present invention may be formulated into powders, granules, tablets, pills, and sugar-coated tablets, capsules, liquids, gels, syrups, slurries, and emulsions by using the method known in the art. For example, tablets or sugar-coated tables may be prepared by mixing an active ingredient with a solid excipient, grinding, and then adding an appropriate adjuvant. For examples of appropriate excipients, it may comprise sugars comprising lactose, dextrose, sucrose, sorbitol, mannitol, xylitol, erythritol and maltitol, starches comprising corn starch, wheat starch, rice starch and potato starch, celluloses comprising cellulose, methyl cellulose, sodium carboxymethylcellulose and hydroxypropylmethylcellulose, and fillers comprising gelatin and polyvinylpyrrolidone. And, if desired, it may comprise cross-linked polyvinylpyrrolidone, agar, alginic acid or sodium alginate as a solutionizer. Further, the pharmaceutical composition for use according to the present invention may comprise anti-coaglutinating agent, lubricant, wetting agents, flavors, emulsifying agents and antiseptics.

In case of parenternal preparations, it comprise injections, creams, lotions, ointments, oils, humectants, gels, and aerosols. The formulation of the above-mentioned is well described in Remington's Pharmaceutical Science, 15th Edition, 1975.

The total effective amount of the pharmaceutical composition for use according to the present invention can be administered to a subject as a single dose, or can be administered using a fractionated treatment protocol, in which the multiple doses are administered over a more prolonged period of time. The pharmaceutical composition for use according to the present invention can be varied in amount of effective component depending on the severity and/or object of disease, but preferably, it may be administered by 0.01 µg/kg to 500 mg/kg, and most preferably 0.1 µg/kg to 100 mg/kg per a day. However, one skilled in the art would know that the concentration of toosendanin or chinaberry tree (Melia azedarach or Melia toosendan) extract to obtain an effective dose in a subject depends on many factors including the age, body weight, health condition, disease severity, diet and excretion of the subject, the route of administration, the number of treatments to be administered, and so forth. In view of these factors, any person skilled in the art can determine the suitable effective dose of toosendanin or chinaberry tree (Melia azedarach or Melia toosendan) extract for preventing or inhibiting dementia. No particular limitation is imposed on the formulation, administration route and administration mode of the pharmaceutical composition for use according to the present invention, as long as the composition shows the effects of the present invention.

The present invention provides a use of toosendanin or a pharmaceutically acceptable salt thereof for preparing an agent for preventing or treating Dementia.

The present invention provides a use of chinaberry tree (Melia azedarach or Melia toosendan) extract for preparing an agent for preventing or treating Dementia.

For preventing or treating Dementia an effective amount of toosendanin or a pharmaceutically acceptable salt thereof is administered to a subject in need thereof.

For preventing or treating dementia an effective amount of chinaberry tree (Melia azedarach or Melia toosendan) extract is administered to a subject in need thereof.

Toosendanin or a pharmaceutically acceptable salt thereof, chinaberry tree (Melia azedarach or Melia toosendan) extract used in the present invention may be administered with an effective amount by various routes including oral, intracutaneous, subcutaneous, intravenous or intramuscular. As used herein, the "effective amount" refers to the amount effective in treating dementia when it is administered to a patient.

As used herein, the "subject" refers to mammals, particularly, animals comprising human and it may be a cell, a tissue or organ originated from the animal. The subject may be patient in need of treatment.

Toosendanin or a pharmaceutically acceptable salt thereof, chinaberry tree (Melia azedarach or Melia toosendan) extract used in the present invention may be administered alone by itself or various formulations which are mentioned above, and preferably it may be administered until effect on dementia is deduced.

### Advantageous Effects

Accordingly, pharmaceutical composition for use according to the present invention including chinaberry tree extract, toosendanin, or its pharmaceutically acceptable salt, as an active ingredient, is very effective in prevention or treatment of dementia. Especially, the composition, for use according to the present invention, is effective in the inhibition of Aβ production and induction of the production promotion of APPα showing a protective effect of a nerve cell. Thus, it is highly effective in prevention or treatment of dementia such as Alzheimer's disease. Accordingly, the pharmaceutical composition for use according to the present invention including chinaberry tree extract, or toosendanin, as an active ingredient, may be used in various fields such as preparation of preventive/treatment drugs for dementia patients or people of ages susceptible to dementia.

### Description of Drawings

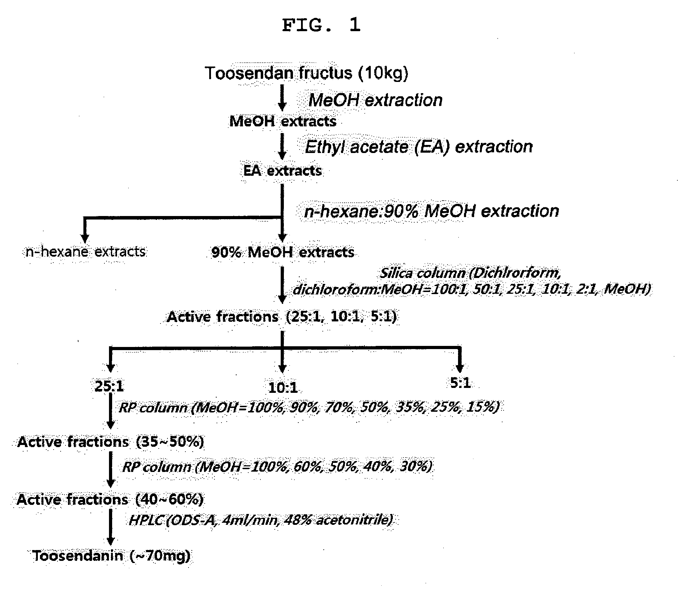

FIG. 1 is a diagram showing a process of obtaining toosendanin according to the present invention, and toosendan fructus' extract and fraction including toosendanin;

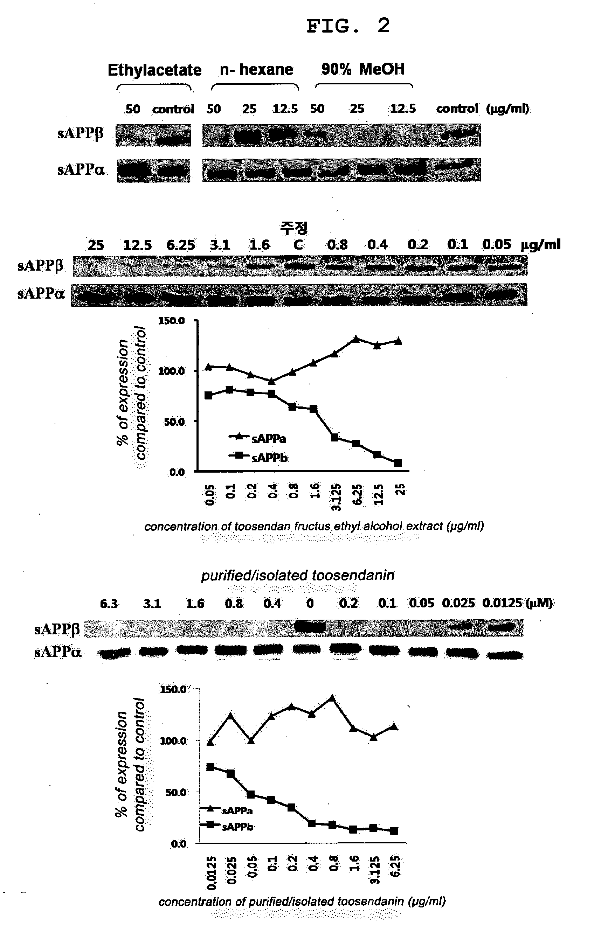

FIG. 2 shows the measurement result of the effect on production of APPα and APPβ (APP metabolites) by toosendanin, and respective organic solvent toosendan fructus extracts including toosendanin, wherein the measurement was carried out by western blot (control, c: a control group not treated with an extract; MeOH: and a group treated with a toosendan fructus methanol extract);

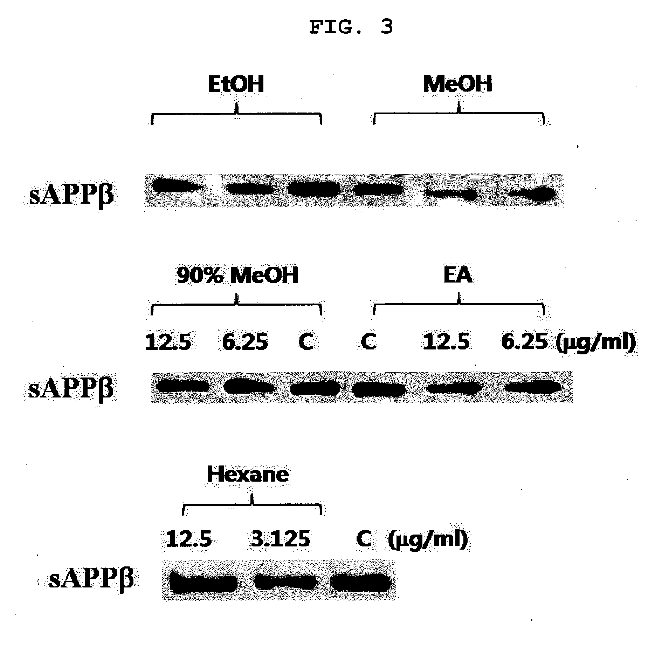

FIG. 3 shows the measurement result of the effect on production of APPβ (APP metabolite) by meliae cortex extracts, wherein the measurement was carried out by western blot (EtOH: a group treated with a meliae cortex ethanol extract; MeOH: a group treated with a meliae cortex methanol extract; EA: a group treated with a meliae cortex ethyl acetate extract; Hexane: a group treated with a meliae cortex n-hexane extract; and C: a control group not treated with an extract);

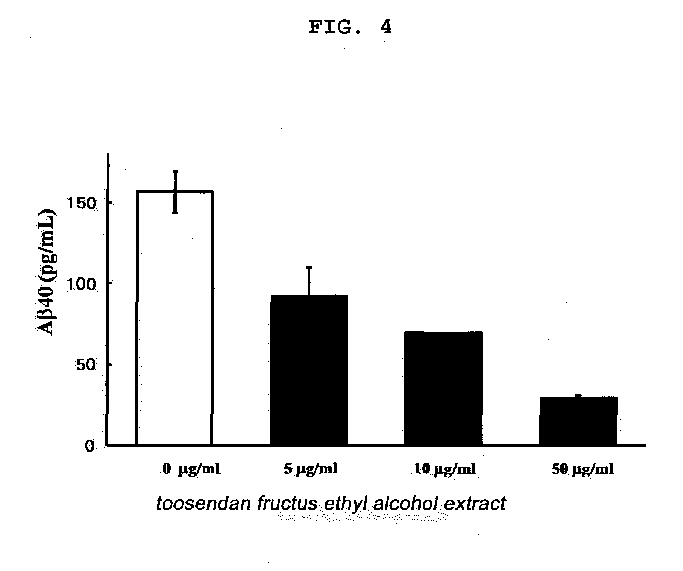

FIG. 4 shows a graph of a measurement result of the amount of Aβ40 when a CHO cell line overexpressing BACE1 and Swedish mutation APP695 was treated with a toosendan fructus ethyl alcohol extract, wherein the measurement was carried out by an Aβ40 quantitative analysis kit in order to determine if the production of Aβ40 was inhibited;

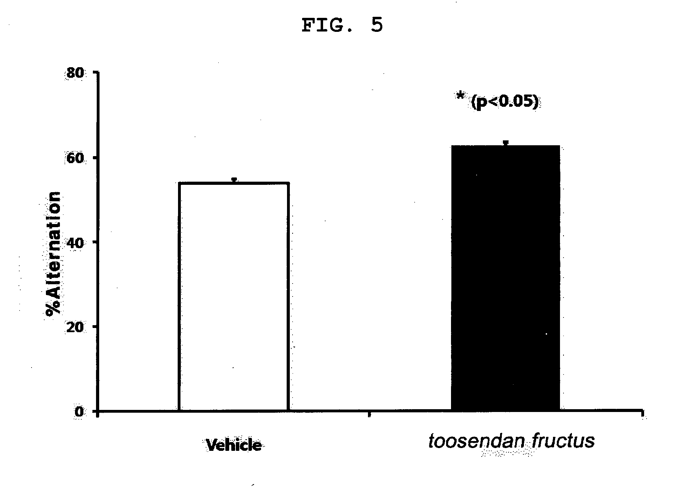

FIG. 5 shows a graph of comparative measurement of spontaneous alternation behavior (%) in a Y-maze test of a dementia rat, wherein the measurement was carried out in order to determine the effect of a toosendan fructus ethyl alcohol extract on memory improvement (% Alternation: alternation behavior (%); Vehicle: a control group administered with only 10% labrasol without an extract; and toosendan fructus: a group administered with a toosendan fructus ethyl alcohol extract);

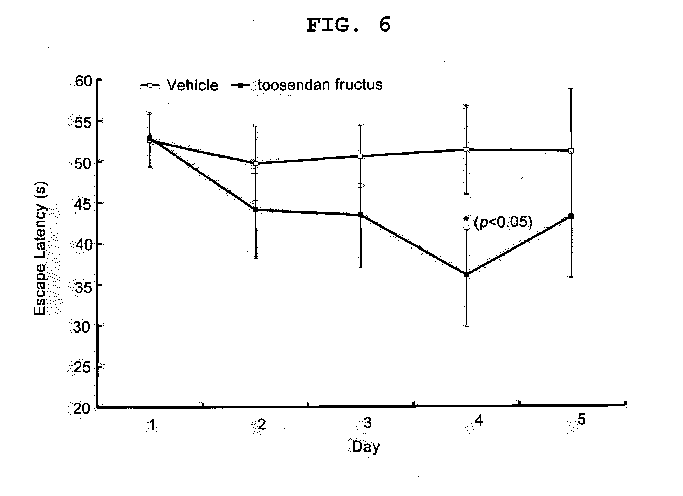

FIG. 6 shows a graph of a comparative measurement result of the time required for finding out a platform in a water maze test of a dementia rat, wherein the measurement was carried out in order to determine the effect of a toosendan fructus ethyl alcohol extract on memory improvement (Escape latency time (s): time required for finding out a platform (s); Vehicle: a control group administered with only 10% labrasol without an extract; and toosendan fructus: a group administered with a toosendan fructus ethyl alcohol extract);

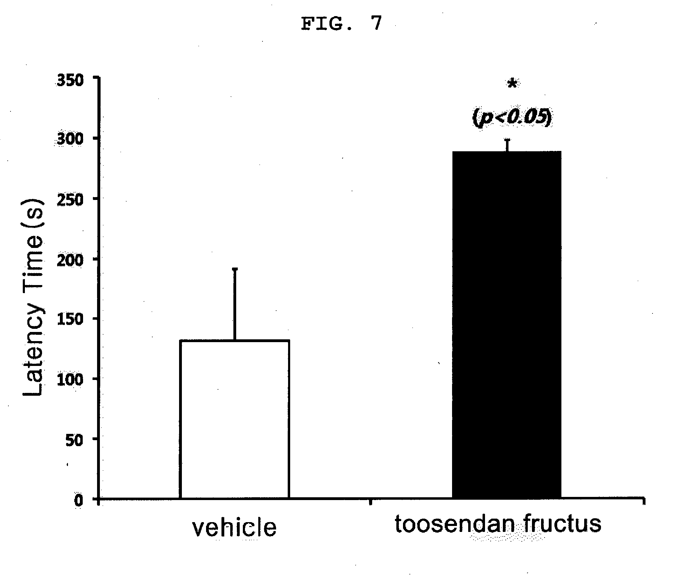

FIG. 7 shows a graph of a measurement result of the time required for entering a dark room in a passive avoidance test of a dementia rat, wherein the measurement was carried out in order to determine the effect of a toosendan fructus ethyl alcohol extract on memory improvement (Retention Latency time (s): time required for entering a dark room (s); Vehicle: a control group administered with only 10% labrasol without an extract; and toosendan fructus: a group administered with a toosendan fructus ethyl alcohol extract);

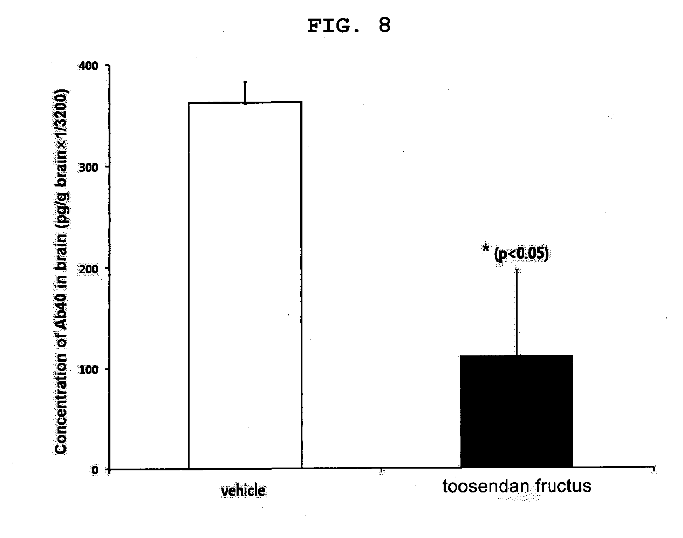

FIG. 8 shows a graph of a measurement result of the amount of Aβ40 in a rat brain tissue wherein the measurement was carried out by an Aβ40 quantitative analysis kit in order to determine if the production of Aβ40 was inhibited by administration of a toosendan fructus ethyl alcohol extract in a brain tissue of a dementia rat;

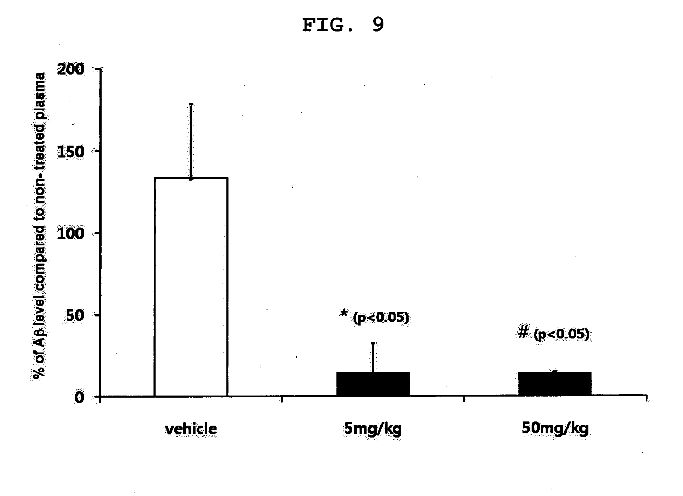

FIG. 9 shows a graph of a measurement result of the amount of Aβ40 in a rat serum wherein the measurement was carried out by an Aβ40 quantitative analysis kit in order to determine if the production of Aβ40 was inhibited in blood of a rat administered with toosendanin (Vehicle: a control group administered with only 20% ethyl alcohol without toosendanin; and 5mg/kg, 50mg/kg: experimental groups administered with toosendanin in a dose of 5mg/kg, and 50mg/kg, respectively;

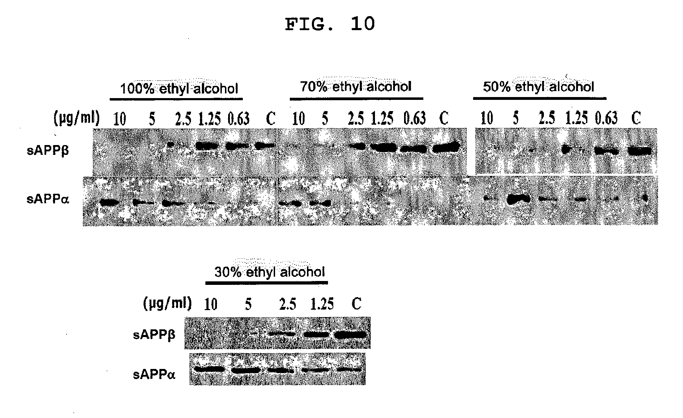

FIG. 10 shows a photograph of a comparative result of the changes in the composition's efficacy for inhibiting APPβ production or promoting APPα production, according to extraction solvents, wherein the result was measured by western blot (100% ethyl alcohol to 30% ethyl alcohol: a group administered with chinaberry tree extract extracted by a mixed solvent having water and ethyl alcohol of respective ratios (%); and C: a control group not treated with an extract); and

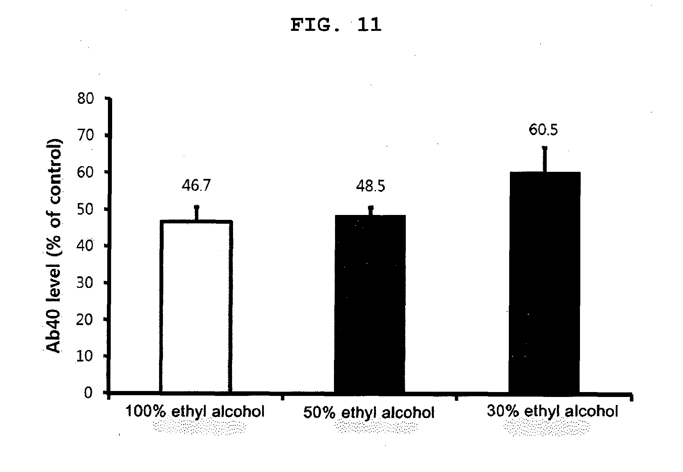

FIG. 11 shows a graph of a measurement result of the changes in the composition's efficacy for inhibiting Aβ production, according to extraction solvents, (100% ethyl alcohol to 50% ethyl alcohol: a group administered with chinaberry tree extract extracted by a mixed solvent having water and ethyl alcohol of respective ratios (%)).

### Mode for Invention

Hereinafter, the present invention will be described in detail with reference to Examples.

However, the following Examples illustrate the invention and are not intended to limit the same.

### <Example 1>

### Extraction and fraction of toosendan fructus and meliae cortex

Finely grinded toosendan fructus (dry-weight: 10kg) was immersed in 50L of methanol for 3 days for extraction. This step was repeated twice. Then, the extract was collected, filtered and vacuum-concentrated. The obtained concentrate was added with 10L of water, suspended, and added with ethyl acetate in the same volume, followed by stirring and settling. Then, an ethyl acetate solvent fraction was collected, and vacuum-concentrated. In order to further fraction the ethyl acetate concentrate, 10L of 90~95% methanol (5~10% water) and 10L of n-hexane were added thereto, followed by stirring and settling. Then, a 90~95% methanol fraction was collected and vacuum-concentrated. Respective fractions obtained in the extraction and fractionation steps were used for activity analysis, and stored at 4°C until their active ingredients were separated. Meliae cortex was also extracted by the same method, and respective solvent extracts were obtained.

### <Example 2>

### Separation of an active ingredient from toosendan fructus sub-fraction

From the concentrate of the 90~95% methanol fraction obtained from Example 1, an active ingredient was separated. The concentrate was fractionated into 8 sub-fractions by silica resin (60 mesh) while the mixing ratio of methanol was gradually increased (from 100% dichloromethane: 0% methanol to 0% dichloromethane: 100% methanol). From these, 3 fractions having activity (dichloromethane: methanol = 25:1, 10:1. 5:1) were secured. The concentrate of the 25:1 fraction having the highest activity was fractionated through octadecyl-silylated silica resin (Biotage FLASH+™ system, C18HS 25+M, 60Å, 40-63µm, FPK0-1107-16046, 10ml/min) while the concentration of MeOH was increased from 15% to 100% by stages. After examination of activities, an active fraction of 35% to 50% was secured. This active fraction was re-fractionated under the same condition by changing the concentration (30~100%) of MeOH. Then, an active fraction (40~60%) was secured, and concentrated. From the concentrate, an active ingredient was purified by using a mixed solvent of acetonitrile and water as a separation solvent (acetonitrile 48%), through high performance liquid chromatography (HPLC) using octadecyl-silylated silica resin (YMC-pack, ODS-A,150×10 mm i.d., 120Å, S-5µm, AA12S05-1510WT) at a flow rate of 4ml/min (see FIG. 1).

The purified active ingredient was identified as toosendanin represented by Formula 1 through structural analysis and molecular weight determination by using an NMR spectroscopy analysis and a mass spectroscopy.

### <Example 3>

### Measurement of content of toosendanin within a toosendan fructus ethyl alcohol extract

In order to carry out an efficacy test of a toosendan fructus ethyl alcohol extract, the content of toosendanin (as an indicator/active ingredient) within the toosendan fructus ethyl alcohol extract was measured. Toosendanin was analyzed by using 30% acetonitrile containing 0.05% TFA (trifluoroacetic acid) as an elution solvent, through HPLC using octadecyl-silylated silica resin (YMC-pack, ODS-A, 150×10 mm I.d., S-5µm) as a column, at a flow rate of 1ml/min at 214nm. The bought toosendanin reference material was diluted to an appropriate concentration, and the area value was measured according to a concentration. Based on this, on a chromatogram of the reference material, an absolute calibration curve of the area-to-concentration ratio was made, and it was determined that the linearity was 0.999. Then, the curve was used for quantification. Toosendanin naturally exists as isomers, and thus was detected (after 15.7 minutes and 20.6 minutes, respectively) under the above described analysis condition. For further exact quantification, a toosendan fructus ethanol extract was suspended in distilled water, solvent-fractionated by ethyl acetate, and concentrated. The concentrate was used for the analysis. When quantitative analysis was carried out by substitution into the calibration curve, it was found that toosendanin was included in an amount of about 1.15% within the toosendan fructus ethyl alcohol extract.

### <Example 4>

### Measurement of cytotoxicity of a toosendan fructus ethyl alcohol extract

The cytotoxicity was measured by an MTT method. As cell lines, HEK293-derived BA-3 cell line (Yeon et al, Peptides, 2007, 28:838-844) to be used for an APPβ production inhibition test, HL60 (human leukemia cell line), and HepG2 (human liver cancer cell line) were used. Cells were plated on 96-well plate at a concentration of 1 × 10⁴/90ul/well, and cultured for 1 day. Each material dissolved in DMSO was diluted by a culture medium so that a final concentration can be 1%, and then each cell line was treated with the material, and cultured for 3 days. After the culture, the state of cells was observed by a microscope. MTT [3-(4,5-dimethylthiazol-2yl)-2,5-diphenyl-2Htetrazolium bromide, 5mg/ml in PBS] solution was added in an amount of 15ul to each well, followed by a reaction for 4 hours at 37°C. Each well was treated with 100ul of lysis buffer(0.01N HCl, 10% SDS), and left at 37°C for 24 hours so that the formed formazan could be completely dissolved. Then, the absorbance was measured at 570/652nm. As a result, BA-3 treated with the toosendan fructus ethyl alcohol extract did not show cytotoxicity even at the highest concentration of 100ug/ml, while the toosendan fructus ethyl alcohol extract slightly induced a cell growth inhibiting effect. Through microscopic observation, cytotoxicity was not found in HepG2 and HL60, treated with the toosendan fructus ethyl alcohol extract, while the cell growth was inhibited. When the toosendan fructus extract inhibited the growths of cells by 50%, its concentrations were 3.2ug/ml and 34ug/ml, respectively.

### <Example 5>

### Analysis of a metabolism inhibiting effect of an amyloid precursor protein (APP) by a toosendan fructus extract and toosendanin in a cell

### <5-1> APPβ production inhibition effect

A HEK293-derived BA-3 cell line overexpressing BACE1(β-secretase) and APP695 was plated on 6-well plate by using DMEM (Dulbecco's Modified Eagle's Medium) including 10% fetal bovin serum (FBS) in such a manner that it occupied 80% or more of the area in each well, and then was cultured for 1 day. After the removal of the culture medium, free DMEM (not including fetal bovine serum) was heated to 37°C, and added in an amount of 0.9ml to each well. Then, toosendanin or a toosendan fructus fraction extracted with each solvent was diluted to an appropriate concentration by 0.1ml free DMEM, and the cell line was treated with these materials, and cultured for 24 hours. 0.2ml of the culture medium treated with each test sample was collected, and subjected to electrophoresis by 8% SDS-PAGE (polyacrylamide gel electrophoresis). Then, AB1560(monoclonal) and Rb53(polyclonal) antibodies (antibodies against APPα and APPβ) were used to measure the amounts of APPα and APPβ within the culture medium through western blotting.

As a result, it was found that methanol/ethyl alcohol/ethyl acetate extracts of the toosendan fructus inhibited the production of APPβ, and promoted the production of APPα. In a test on the effect on APPβ production inhibition and APPα production promotion according to dilution concentrations of the ethyl alcohol extract, when the treatment concentration of the extract was 6.25ug/ml, the production of APPβ was reduced to 27.7%, and the production of APPα was increased to 131.3%, with respect to a non-treated group (Control, 100%). On the other hand, even when the treatment concentration of the extract was lowest (less than 0.05µg/ml), a concentration-dependent inhibition effect of APPβ was achieved and the production of APPα was promoted. Also, it was found that purified toosendanin even at a concentration of 12.5nM or less inhibited the production of APPβ while promoting the production of APPα (at 0.8uM, production of APPβ reduced to 17.8%, and production of APPα increased to 141.5%, with respect to a non-treated group). Thus, it can be determined that these materials very strongly inhibit APP metabolism promoting Aβ production (see FIG. 2).

Extracts (ethyl alcohol, methanol, ethyl acetate, and 90% methanol) of meliae cortex (bark of chinaberry) were obtained under the same organic solvent extraction condition of toosendan fructus. Then, their effects on APPβ production reduction were tested. As a result, every extract showed an APPβ production reducing effect at 12.5 - 6.25µg/ml although the effect was lower than that of the toosendan fructus extract (see FIG. 3).

### <5-2> Aβ production inhibition effect by a toosendan fructus extract

BACE1 (GenBank accession No; AF190725) and Swedish mutation APP695 (GenBank accession No; MIM: 104760.008) genes were introduced into vectors of pcDNA3.1/myc-His(Invitrogen, USA) and pcDNA3.1/Hygro(Invitrogen, USA), respectively, and introduced into CHO cell lines. Then, by using a cell line overexpressing BACE1 and Swedish mutation APP695 proteins, it was tested if the toosendan fructus extract inhibited the production of Aβ40. The CHO cell lines were plated on a 6-well plate by using DMEM including 10% FBS (fetal bovin serum) in such a manner that they were included in an appropriate amount in each well, and cultured for 1 day. After the removal of the culture medium, free DMEM (not including fetal bovine serum) was heated to 37°C, and added in an amount of 0.9ml to each well. Then, a toosendanin-containing toosendan fructus extract was diluted to an appropriate concentration by 0.1ml free DMEM, and the cell lines were treated with the extract, and cultured for 24 hours. Then, the culture medium was separated, and the quantitative analysis was carried out by an Aβ40 quantitative analysis kit (Human β Amyloid (1-40) colorimetric ELISA kit, BioSource, CA) of BioSource (CA, USA).

As a result, it was observed that the toosendan fructus ethyl alcohol extract (10ug/ml or less) showed an Aβ40 production inhibition effect of 50% or more (see FIG. 4).

### <Example 6>

### Toxicity test on a toosendan fructus ethyl alcohol extract

In order to determine if oral-administration of the toosendan fructus ethyl alcohol extract containing toosendanin causes toxicity, toxicity tests on a single dose and 4-week multiple doses were carried out.

In the single-dose toxicity test, each group including 5 male ICR rats (aged 6 weeks) was orally administered with a toosendan fructus ethyl alcohol extract as a single dose in an amount of 750~3,000mg/kg. It was observed for 2 weeks if the experimental animals survived, and then through euthanasia and autopsy, abnormal findings in organs were observed. It was found that the administration of the extract in an amount of 1,500mg/kg or more caused toxicity, because some of rats died. On the other hand, it was found that the administration of the extract in an amount of 1,000mg/kg or less did not cause toxicity because no rats died and no abnormal findings in organs were observed.

In the multiple-dose toxicity test, each group including 6 male ICR rats (aged 6 weeks) was orally administered with a toosendan fructus ethyl alcohol extract in an amount of 125~1, 000mg/kg once a day for 26 days. A weight change, general symptoms, and mortality of the experimental animals were observed, and through euthanasia and autopsy, abnormal findings in organs were observed. As a result of the test, it was found that a maximum tolerance dose (MTD) was 750~1,000mg/kg, and a no-observed-adverse effect level (NOAEL) was 500mg/kg.

### <Example 7>

### An effect on memory and cognitive function improvement by a toosendan fructus ethyl alcohol extract in a dementia animal model

A toosendan fructus ethyl alcohol extract suspended in 10% labrasol was repeatedly orally administered to 6 dementia rats (transformed to produce Swedish mutation APP) in an amount of 300mg/kg (5 times per week) for 3 months. Then, a Y maze test, a water maze test, and a passive avoidance test were carried out, and it was analyzed if a memory improving effect was achieved unlike a vehicle group (administered with 10% labrasol, 5 rats).

### <7-1> a Y maze test

The test was performed by using a Y maze measurement apparatus with 3 arms (each arm: 25(L) x 14(H) x 5(W) (cm)) forming a letter Y (arm) at uniform angles. First, the head of an experimental animal was pointed toward one arm end of the Y maze, and the animal was allowed to freely run around the arms for 8 minutes. The case where the dementia rat's hind leg entered the arm was recognized as an arm entry. The movement was recorded as alternation. Then, 3 sequential entries were recognized as an actual alternation, and were given 1 point. Then, spontaneous alternation behavior (%) was obtained by the percentage of the actual alternation to possible maximum alternation (= total alternation - 2).

As a result, the group administered with the toosendanin-containing toosendan fructus ethyl alcohol extract showed a spontaneous alternation behavior of 62.6± 3.1%, and the vehicle group (10% labrasol) showed 53.9± 3.4%. Thus, it was found that the administration of the toosendan fructus ethyl alcohol extract showed a memory improving effect of about 16.1% with reliability of 95% or more (p<0.05) (see FIG. 5).

### <7-2> a water maze test

A reference memory test for the water maze test was carried out after rats were freely left to swim and adapted in a water tank with no platform for 60 seconds, one day before the start of the test. In the test, the time (escape latency, unit: second) required for finding out and mounting the platform immersed in water was measured 4 to 5 times per day, for 5 days, and a maximum tolerance time was limited to 60 seconds. Until the second day of the test, when a rat could not find out the position of the platform, it was guided to find out the platform within 60 seconds (limit time). Then, once having mounted the platform, it was allowed to stay for 10 seconds.

After 24 hours from the end of the reference memory test, the platform within the water tank was removed, and rats were allowed to freely swim for 60 seconds. Then, a probe test was carried out while a rat's staying time in the position (where the platform existed in past days) was measured.

As a result, in the reference memory test, a vehicle group did not show improvement in the time required for finding out the platform during the test period while a group administered with a toosendan fructus ethyl alcohol extract found out the platform in a shorter time than the vehicle group, from the second day of the test. Then, from the fourth day of the test, the difference between the group administered with the extract and the vehicle group was maximized (see FIG. 6). In the probe test, the extract-administered group showed a stay ratio of 33.0± 3.2% in the position (where the platform existed in past days) while the vehicle group showed a ratio of 14.0± 5.8%. It was found that the extract-administered group stayed for a 2.35 times or more (p<0.05) longer period with statistical significance.

### <7-3> a passive avoidance test

The passive avoidance test was carried out by using a shuttle box having 2 sections sectioned by a guillotine door. One section was brightened by lighting while the other section was darkened with a covering of black cloth without lighting. On the first day, a dementia rat was left in the bright room with a search time of 30 seconds, and then the guillotine door was opened so that the rat can enter the dark room. Then, an acquisition latency time required for entering the dark room was measured. Once the rat entered the dark room, the guillotine door was closed, and an electric shock of 0.5mA was applied to the rat for 3 seconds through a lattice floor so that it can remember the electric shock. After 24 hours, the dementia rat was left in the bright room with a search time of 30 seconds and then the guillotine door was opened. Then, a retention latency time required for entering the dark room (all feet of the rat) was measured (maximum of 300 seconds). It was determined that as the retention latency time increases, the memory on passive avoidance through learning can be well maintained.

When the retention latency time required for entering the dark room after the electric shock learning was measured, a group administered with a toosendan fructus ethyl alcohol extract showed a retention latency time of 288.8± 9.2 seconds, and a vehicle group showed a retention latency time of 131.8± 59.6 seconds. In other words, it was found that the extract-administered group showed a memory improving effect on passive avoidance of 220% or more with a reliability of 95% or more (see FIG. 7). Accordingly, it was found that the composition for use according to the present invention has a significant effect on the treatment of memory/cognition dysfunction caused by dementia.

### <Example 8>

### Aβ inhibiting effect by toosendan fructus ethyl alcohol extract and toosendanin in living body

### <8-1> Aβ inhibiting effect by a toosendan fructus ethyl alcohol extract in a transformed dementia rat

After the end of the memory test, a brain of the transformed rat was taken out and extracted. Then, within a brain tissue, a change of Aβ was measured. The extracted brain was weighed, and added with a cooled solution A (5M guanidine HCl, 50mM Tris HCl) in a volume 8 times greater with respect to the weight. Then, the brain tissue was grinded and homogenized by a homogenizer, and left at room temperature for 3~4 hours, followed by centrifugation (16,000× g, 20 minutes, 4°C). A supernatant was collected so as to extract the brain tissue. The collected supernatant was diluted 3,200 times by a solution B(Phsophate buffered saline, 5% BSA, 0.03% Tween-20, pH7.4, protease inhibitor cocktail), and Aβ40 was quantified by using an Aβ40 quantitative analysis kit of BioSource (CA, USA).

As a result, when the value of Aβ40 with respect to the weight of brain tissue diluted 3,200 times was measured, the value of a vehicle group was 362.5± 20.7pg/ml, and the value of a toosendan fructus ethyl alcohol extract-administered group was 111.9± 84.6pg/ml. In other words, the administration of the toosendan fructus ethyl alcohol extract reduced Aβ40 by about 69% (see FIG. 8).

### <8-2> Aβ inhibiting effect by toosendanin in a rat

Before administration of toosendanin, blood was secured. Then, toosendanin suspended in 20% ethyl alcohol was orally administered in doses of 5 and 50mg/kg. After two hours, the blood was collected, and the concentration change of Aβ within the blood before/after administration was measured. The collected blood was centrifuged (16,000×g, 20 minutes, 4°C), and Aβ40 within the collected serum was quantified by using an Aβ40 quantitative analysis kit (Mouse/Rat Amyloidβ (1-40) High Specific Assay Kit, IBL, Japan).

As a result, a vehicle group showed an increase of Aβ40 by about 40% through the administration, without statistical significance. Also, it was found that the administration of toosendanin (5mg/kg) inhibited Aβ40 by 85% or more. From this result, it is expected that if toosendanin is administered in a lower dose, the production of Aβ40 can be inhibited (see FIG. 9). Accordingly, it was determined that the composition for use according to the present invention is highly effective in prevention or treatment of dementia by inhibiting the production of Aβ.

### <Example 9>

### toxicity and efficacy test according to an extraction solvent

In order to obtain a lowest-toxicity extract capable of maintaining the efficacy of the composition for use according to the present invention, toosendan fructus was extracted by varying the content of ethyl alcohol in an extraction solvent, and toxicity and efficacy tests were carried out.

### <9-1> Preparation of a toosendan fructus ethyl alcohol extract

10kg of grinded toosendan fructus 10kg was immersed in 50L of ethyl alcohol (100%, 70%, 50% and 30%) for 3 days for extraction. Then, the extract was filtered, vacuum-concentrated, and freeze-dried so as to obtain a toosendan fructus ethyl alcohol extract.

### <9-2> repetitive toxicity test on a toosendan fructus ethyl alcohol extract

One vehicle group and three administration groups according to ethyl alcohol concentrations were prepared, in which each group included 6 male ICR rats (aged 6 weeks).

The toosendan fructus ethyl alcohol extract obtained from Example 9-1 was dissolved in 10% labrasol (Gate Fosse Inc., France) as an excipient, and orally administered to a rat once per day. Ethyl alcohol extracts with respective concentrations were administered to each group in doses of 1000, 1500, and 2000mg/kg.

On the day of administration, general symptoms were observed with an interval of one hour during 6 hours from the administration. Then, during the days after administration, general symptoms were observed and recorded once a day. Twice a week, the animal was weighed on the weighting day. After the end of administration, the animal was fasted overnight, and then its blood was collected, and subjected to a blood biochemical examination. After the blood collection, the animal was subjected to autopsy, and abnormal findings in main organs were observed.

For animals in each group, an average weight according to measurement days and mortality were recorded, and then, MTD and NOAEL of the test sample were obtained. In order to determine if there is a difference in an average weight and a blood biochemical value between a control group and administration groups, Dunnett's test as a multiple comparison method was carried out, and a critical region for the test was set as 5% (*P < 0.05).

MTD refers to a minimum dose of a test material administered to a test animal which will cause toxicity symptoms having no effect on death, and a weight increase/decrease by 10% or less with respect to a control group. NOAEL denotes the highest exposure level in a toxicity test, at which there is no harmful effect of the administered material in the long term.

As a result, as noted in Table 1, as the content of ethyl alcohol was decreased in the extraction, the NOAEL increased. Also, it was found that in the 30% ethyl alcohol extract, there was no toxicity even in the maximum administration dose of 2,000mg/kg.

**Table 1**

| MTD and NOAEL results according to extraction solvents | | |
|---|---|---|
| Ethyl alcohol extract | MTD(mg/kg) | NOAEL(mg/kg) |
| 100% Ethyl alcohol | <1,000 | 500-750 |
| 70% Ethyl alcohol | >2,000 | <1,000 |
| 50% Ethyl alcohol | >2,000 | 1,000-1,500 |
| 30% Ethyl alcohol | >2,000 | >2,000 |

### <9-3> efficacy comparison test according to the kinds of extraction solvents of a toosendan fructus ethyl alcohol extract

On the toosendan fructus ethyl alcohol extract obtained from Example 9-1, an APPβ production reducing effect, an APPα production promoting effect, and an Aβ production inhibiting effect were measured in the same manner as described in Example 5.

As a result, as shown in FIG. 10, it was found that the used chinaberry extract of the present invention obtained by 100%, 70%, 50% and 30% ethyl alcohols as an extraction solvent are excellent in both an APPβ production reducing effect and an APPα production promoting effect. Also, as shown in FIG. 11, it was found that an Aβ inhibiting effect was generally high although the extraction by 30% ethyl alcohol reduced the efficacy by about 14%.

Accordingly, when the used chinaberry extract of the present invention was extracted by ethyl alcohol as an extraction solvent, it is possible to achieve overall high efficacy irrespective of ethyl alcohol concentration. Especially, it was found that when as the content of ethyl alcohol used as an extraction solvent was adjusted in a range of 30% to 50%, it is possible to maintain the efficacy and to highly reduce the toxicity in the used chinaberry tree extract of the present invention.

Although formulation examples of a pharmaceutical composition for use according to the present invention including the compound of the present invention were described, they specifically illustrate the invention and are not intended to limit the same.

### <Example of Preparation 1>

### Preparation of powder

| | |
|---|---|
| toosendan fructus extract | 20 mg |
| lactose | 100 mg |
| talc | 10 mg |

After mixing the above ingredients and filling in an airtight pouch and they were prepared into powder.

### <Example of Preparation 2>

### Preparation of tablet

| | |
|---|---|
| toosendan fructus extract | 10 mg |
| corn starch | 100 mg |
| lactose | 100 mg |
| magnesium stearate | 2 mg |

After mixing the above ingredients and performing direct compression and they were prepared into tablet according to a well known method.

### <Example of Preparation 3>

### Preparation of capsule

| | |
|---|---|
| Meliae cortex extract | 10 mg |
| crystallized cellulose | 3 mg |
| lactose | 14.8 mg |
| magnesium stearate | 0.2 mg |

After mixing the above ingredients and filling into gelatin capsule and they were prepared into capsule according to a well known method.

### <Example of Preparation 4>

### Preparation of Injection

Active ingredient is dissolved into distilled water for injection according to a well known method, and adjust pH to 7.5 and the below ingredient were dissolved in distilled water for injection. Then filled in 2 ml of ampoule, sterilized and injection were prepared.

| | |
|---|---|
| toosendan fructus extract | 1 mg |
| mannitol | 180 mg |
| water for injection | 2793 mg |
| Na₂HPO₄12H₂O | 26 mg |

One ampoule(2 ml) per the above ingredients is prepared according to a well known method.

### <Example of Preparation 5>

### Preparation of Fluid

| | |
|---|---|
| toosendan fructus extract | 2 mg |
| isomerized glucose syrup | 10 g |
| mannitol | 5 g |
| appropriate distilled water | |

According to a well known method, dissolve all ingredients into distilled water and add appropriate lemon flavor and then mix and adjust the volume to 100 ml by adding distilled water. After adjustment, it is filled into brown bottle and sterilized.

### Industrial Applicability

As can be seen foregoing, a pharmaceutical composition for use according to the present invention comprising chinaberry tree extract, toosendanin, or a pharmaceutically acceptable salt thereof as an active ingredient is very effective in prevention or treatment of dementia. Especially, the composition for use according to the present inventionis effective in the inhibition of Aβ production and induction of the production promotion of APPα showing a protective effect of a nerve cell. Thus, pharmaceutical composition for use according to the present invention comprising chinaberry tree extract, toosendanin, or a pharmaceutically acceptable salt thereof as an active ingredient, is highly effective in various fields such as preparation of preventive/treatment drugs for dementia patients or people of ages susceptible to dementia.

## Claims

1. A composition for use in preventing or treating dementia comprising toosendanin represented by the formula 1 or a pharmaceutically acceptable salt thereof as an active ingredient.

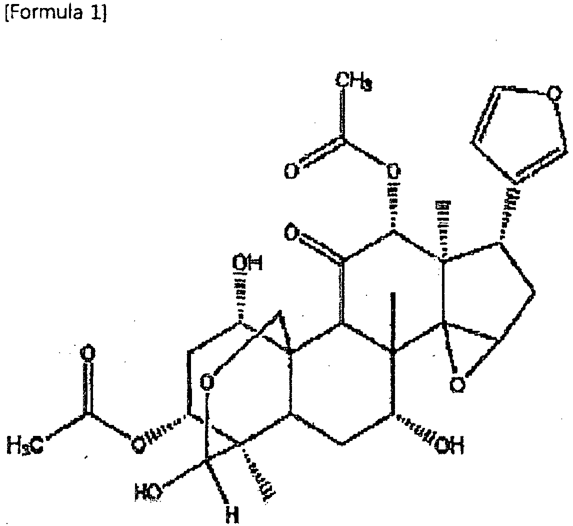

2. The composition for use in preventing or treating dementia according to claim 1, wherein the dementia is selected from the group consisting of Alzheimer dementia, cerebrovascular dementia, senile dementia, and dementia by head injuries.

3. Use of toosendanin represented by the formula 1 or a pharmaceutically acceptable salt thereof for preparing an agent for preventing or treating dementia.

4. The use of claim 3, wherein the dementia is selected from the group consisting of Alzheimer dementia, cerebrovascular dementia, senile dementia, and dementia by head injuries.

5. A composition for use in preventing or treating dementia comprising chinaberry tree (Melia azedarach or Melia toosendan) extract as an active ingredient.

6. The composition for use in preventing or treating dementia according to claim 5, wherein the chinaberry tree is toosendan fructus (Chinaberry's fruit) or Meliae cortex (velamen or bark of chinaberry).

7. The composition for use in preventing or treating dementia according to claim 5, wherein the extract is extracted with ethyl acetate or water, C1 to C4 alcohol or a mixed solvent thereof.

8. The composition for use in preventing or treating dementia according to of claim 5, wherein the dementia is selected from the group consisting of Alzheimer dementia, cerebrovascular dementia, senile dementia, and dementia by head injuries.

9. Use of chinaberry tree (Melia azedarach or Melia toosendan) extract for preparing an agent for preventing or treating dementia.

10. The use of claim 9, wherein the dementia is selected from the group consisting of Alzheimer dementia, cerebrovascular dementia, senile dementia, and dementia by head injuries.

## Patentansprüche

1. Zusammensetzung für die Verwendung zur Prävention oder Behandlung von Demenz, umfassend Toosendanin, dargestellt durch die Formel 1 oder ein pharmazeutisch annehmbares Salz desselben als ein Wirkstoff.

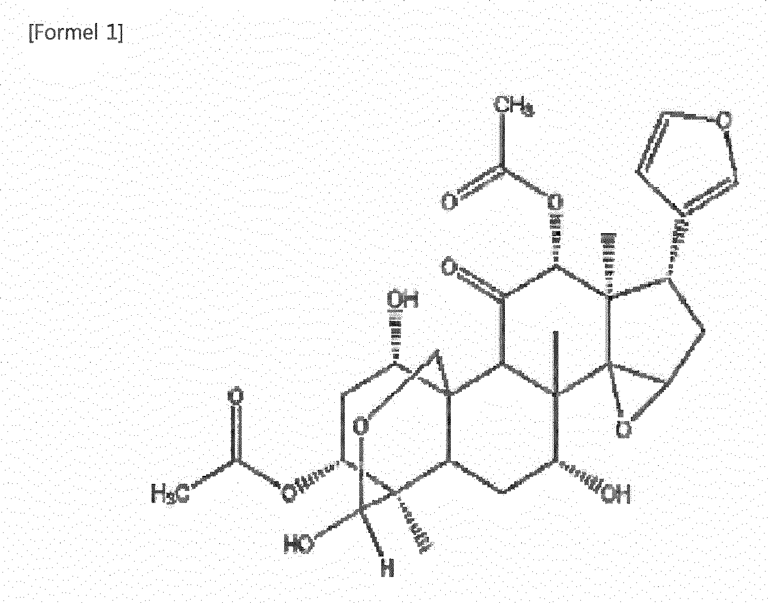

2. Zusammensetzung für die Verwendung zur Prävention oder Behandlung von Demenz nach Anspruch 1, wobei die Demenz aus der Gruppe ausgewählt wird, die aus Alzheimer-Demenz, zerebrovaskulärer Demenz, Altersdemenz und durch Kopfverletzungen verursachte Demenz besteht.

3. Verwendung von Toosendanin, dargestellt durch die Formel 1 oder ein pharmazeutisch annehmbares Salz desselben für die Zubereitung eines Mittels zur Prävention oder Behandlung von Demenz.

4. Verwendung nach Anspruch 3, wobei die Demenz aus der Gruppe ausgewählt wird, die aus Alzheimer-Demenz, zerebrovaskulärer Demenz, Altersdemenz und durch Kopfverletzungen verursachte Demenz besteht.

5. Zusammensetzung für die Verwendung zur Prävention oder Behandlung von Demenz, umfassend Zedrachbaum - (Melia azedarach oder Melia toosendan) Extrakt als einen Wirkstoff.

6. Zusammensetzung für die Verwendung zur Prävention oder Behandlung von Demenz nach Anspruch 5, wobei der Zedrachbaum Toosendan Fructus (Zedrachbaumfrucht) oder Meliae cortex (Wurzelhülle oder Rinde des Zedrach) ist.

7. Zusammensetzung für die Verwendung zur Prävention oder Behandlung von Demenz nach Anspruch 5, wobei der Extrakt mit Ethylacetat oder Wasser, C1- bis C4-Alkohol oder einem gemischten Lösungsmittel derselben extrahiert wird.

8. Zusammensetzung für die Verwendung zur Prävention oder Behandlung von Demenz nach Anspruch 5, wobei die Demenz aus der Gruppe ausgewählt wird, die aus Alzheimer-Demenz, zerebrovaskulärer Demenz, Altersdemenz und durch Kopfverletzungen verursachte Demenz besteht.

9. Verwendung des Zedrachbaum - (Melia azedarach oder Melia toosendan) Extrakts für die Zubereitung eines Mittels zur Prävention oder Behandlung von Demenz.

10. Verwendung nach Anspruch 9, wobei die Demenz aus der Gruppe ausgewählt wird, die aus Alzheimer-Demenz, zerebrovaskulärer Demenz, Altersdemenz und durch Kopfverletzungen verursachte Demenz besteht.

## Revendications

1. Une composition pour l'utilisation lors de la prévention ou du traitement de la démence comprenant de la toosendanine représentée par la formule 1 ou par un sel de celle-ci acceptable d'un point de vue pharmaceutique comme un ingrédient actif.

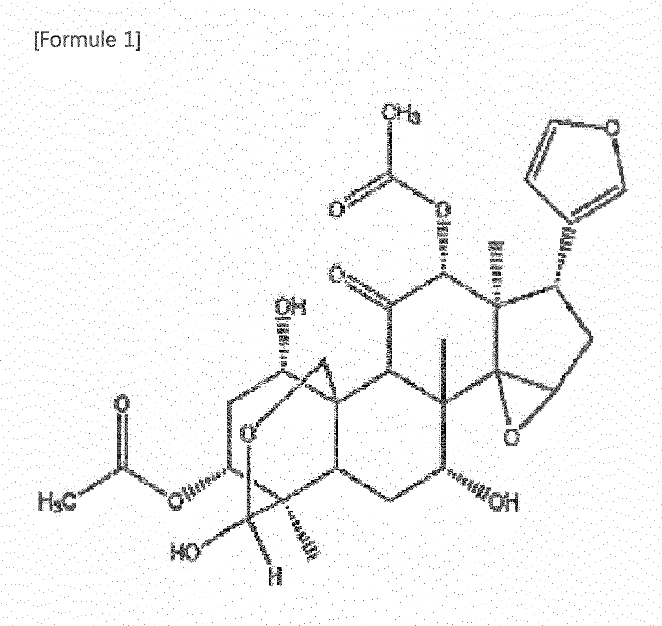

2. La composition pour l'utilisation lors de la prévention ou du traitement de la démence selon la revendication 1, la démence étant sélectionnée parmi un groupe comprenant la démence d'Alzheimer, la démence vasculaire, la démence sénile et la démence suite à des blessures à la tête.

3. Utilisation de la toosendanine représentée par la formule 1 ou par un sel de celle-ci acceptable d'un point de vue pharmaceutique pour préparer un agent en vue de la prévention ou du traitement de la démence.

4. L'utilisation de la revendication 3, la démence étant sélectionnée parmi un groupe comprenant la démence d'Alzheimer, la démence vasculaire, la démence sénile et la démence suite à des blessures à la tête.

5. Une composition pour l'utilisation lors de la prévention ou du traitement de la démence comprenant de l'extrait de mélia azedarach (Melia azedarach ou Melia toosendan) comme ingrédient actif.

6. La composition pour l'utilisation lors de la prévention ou du traitement de la démence selon la revendication 5, le mélia azedarach étant toosendan fructus (fruit du mélia azedarach) ou Meliae cortex (vélamen ou écorce du mélia azedarach).

7. La composition pour l'utilisation lors de la prévention ou du traitement de la démence selon la revendication 5, l'extrait étant obtenu par extraction à l'aide d'acétate d'éthyle ou d'eau, d'un alcool C1-C4 ou d'un solvant mixte de ceux-ci.

8. La composition pour l'utilisation lors de la prévention ou du traitement de la démence selon la revendication 5, la démence étant sélectionnée parmi un groupe comprenant la démence dAlzheimer, la démence vasculaire, la démence sénile et la démence suite à des blessures à la tête.

9. L'utilisation d'extrait de mélia azedarach (Melia azedarach ou Melia toosendan) pour la préparation d'un agent en vue de la prévention ou du traitement de la démence.

10. L'utilisation de la revendication 9, la démence étant sélectionnée parmi un groupe comprenant la démence dAlzheimer, la démence vasculaire, la démence sénile et la démence suite à des blessures à la tête.
